(19) Europäisches Patentamt
European Patent Office
Office européen des brevets

(11) Veröffentlichungsnummer: **0 113 640**
**B1**

(12) # EUROPÄISCHE PATENTSCHRIFT

(45) Veröffentlichungstag der Patentschrift: **23.05.90**

(21) Anmeldenummer: **83810576.5**

(22) Anmeldetag: **08.12.83**

(51) Int. Cl.[5]: **C 07 D 249/08,**
C 07 D 233/60,
A 01 N 43/653, A 01 N 43/50
// C07D303/08, C07D303/22,
C07C39/00

(54) **1-Azolyl-2-aryl-3-fluoralkan-2-ole als Mikrobizide.**

(30) Priorität: **14.12.82 CH 7269/82**

(43) Veröffentlichungstag der Anmeldung:
**18.07.84 Patentblatt 84/29**

(45) Bekanntmachung des Hinweises auf die Patenterteilung:
**23.05.90 Patentblatt 90/21**

(84) Benannte Vertragsstaaten:
**AT BE CH DE FR IT LI LU NL SE**

(56) Entgegenhaltungen:
**EP-A- 47 594**
**EP-A-0 017 080**
**EP-A-0 104 734**
**EP-A-0 117 100**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Sturm, Elmar, Dr.**
**Klusstrasse 66**
**CH-4147 Aesch (CH)**
Erfinder: **Meyer, Alfred, Dr.**
**St. Galler-Ring 220**
**CH-4054 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 113 640 B1

**Beschreibung**

Die vorliegende Erfindung betrifft neue, substituierte 1-Azolyl-2-aryl-3-fluor-alkan-2-ole und deren Ether nachstehender Formel I sowie deren Säureadditionssalze, quaternären Azoliumsalze und Metallkomplexe. Die Erfindung betrifft ferner die Herstellung dieser Substanzen sowie mikrobizide Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von schädlichen Mikroorganismen, vorzugsweise pflanzenschädigenden Pilzen.

Bei den erfindungsgemässen Verbindungen handelt es sich um solche der allgemeinen Formel I:

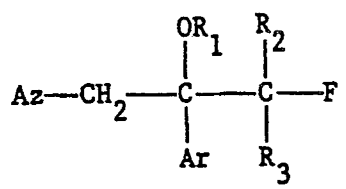

(I)

worin

Az für 1H-1,2,4-Triazol, 4H-1,2,4-Triazol oder 1H-Imidazol steht;

Ar einen unsubstituierten oder substituierten aromatischen Rest aus der Reihe Phenyl, Biphenyl, Phenoxyphenyl und Naphthyl bedeutet;

$R_1$ für Wasserstoff, $C_1$—$C_4$-Alkyl, $C_3$—$C_5$-Alkenyl oder Benzyl steht;

$R_2$ Wasserstoff, Fluor oder $C_1$—$C_6$-Alkyl bedeutet; und

$R_3$ Wasserstoff, Fluor, $C_1$—$C_6$-Alkyl, $C_1$—$C_6$-Haloalkyl, $C_1$—$C_6$-Alkoxy, $C_1$—$C_6$-Alkylthio, Phenyl, Phenoxy, Phenylthio oder $C_3$—$C_7$-Cycloalkyl steht,

wobei jeder aromatische Substituent oder aromatische Anteil eines Substituenten unsubstituiert oder ein- oder mehrfach durch Halogen, $C_1$—$C_4$-Alkyl, $C_1$—$C_4$-Alkoxy, $C_1$—$C_4$-Haloalkyl, Nitro und/oder Cyano substituiert ist, mit der Maßgabe, dass $R_2$ und $R_3$ nicht gleichzeitig Fluor sein dürfen; unter Einschluss der Säureadditionssalze, quaternären Azoliumsalze und Metallkomplexe.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl usw. sowie ihre Isomeren, wie z.B. Isopropyl, Isobutyl, tert.-Butyl, Isopentyl usw. Haloalkyl steht für einen einfach bis perhalogenierten Alkylsubstituenten, wie z.B. $CHCl_2$, $CHF_2$, $CH_2Cl$, $CCl_3$, $CH_2F$, $CH_2CH_2Cl$, CHBr usw., insbesondere $CF_3$. Unter Halogen soll hier und im folgenden Fluor, Chlor, Brom oder Jod, vorzugsweise Fluor, Chlor oder Brom, verstanden werden. Alkenyl bedeutet z.B. Propenyl-(1), Allyl, Butenyl-(1), Butenyl-(2) oder Butenyl-(3). Naphthyl bedeutet α- oder β-Naphthyl.

Die vorliegende Erfindung betrifft somit die freien Verbindungen der Formel I, deren Säureadditionssalze, quaternären Azoliumsalze und Metallkomplexe. Die freien Verbindungen, insbesondere die 1H-1,2,4-Triazolderivate im Umfang der Formel I sind bevorzugt.

Beispiele salzbildender Säuren sind anorganische Säuren: Halogenwasserstoffsäurenwie Fluorwasserstoffsäure, Chlorwasserstoffsäure, Bromwasserstoffsäure oder Jodwasserstoffsäure sowie Schwefelsäure, Phosphorsäure, Phosphorige Säure, Salpetersäure und organische Säuren wie Essigsäure, Trifluoressigsäure, Trichloressigsäure, Propionsäure, Ameisensäure, Benzolsulfonsäure, p-Toluolsulfonsäure oder Methansulfonsäure.

Metallkomplexe der Formel I bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Zirkonium, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d.h. sie können ein oder mehrere organische Molekülanteile als Liganden enthalten. Komplexe mit den Metallen Kupfer, Zink, Mangan, Zinn und Zirkonium sind bevorzugt.

Triazol-phenyl-alkanol-Derivate sind bereits verschiedentlich bekannt. So sind derartige Verbindungen in den europäischen Patentanmeldungen 0 047 594 und 0 104 734 als fungizide Wirkstoffe vorgeschlagen worden.

Die Verbindungen der Formel I sind bei Raumtemperatur stabile Oele, Harze oder überwiegend Feststoffe, die sich durch sehr wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen, dabei sind die Triazolylmethylderivate im Umfang der Formel I bevorzugt. Die erfindungsgemässen Wirkstoffe der Formel I zeichnen sich bei niederigen Anwendungskonzentrationen durch eine sehr gute Pflanzenfungizide Wirkung und problemlose Anwendung aus. Darüberhinaus zeigen sie auch eine wuchsregulierende, insbesondere wuchshemmende Wirkung, vor allem bei tropischen

Bodenbedecker-Pflanzen (Cover-crops).

Aufgrund ihrer ausgeprägten mikrobiziden insbesondere pflanzenfungiziden Wirkung sind folgende Substanzgruppen bevorzugt:

Verbindungen der Formel I worin Az für 1H-1,2,4-Triazol oder 1H-Imidazol steht; Ar einen unsubstituierten oder substituierten aromatischen Rest aus der Reihe Phenyl, Biphenyl oder Phenoxyphenyl bedeutet, $R_1$ für Wasserstoff steht; $R_2$ Wasserstoff, Fluor oder $C_1$—$C_3$-Alkyl bedeutet; und $R_3$ Wasserstoff, Fluor, $C_1$—$C_4$-Alkyl, $C_1$—$C_3$-Haloalkyl, $C_1$—$C_3$-Alkoxy, $C_1$—$C_3$-Alkylthio, Phenyl, Phenyloxy oder Phenylthio bedeutet, wobei jeder Phenylteil unsubstituiert oder durch Fluor, Chlor, Brom, Methyl, Methoxy, $CF_3$, $NO_2$ und/oder Cyano substituiert ist; unter Einschluss der Säureadditionssalze, quaternären Azoliumsalze und Metallkomplexe.

Innerhalb dieser Gruppe sind diejenigen Verbindungen der Formel I besonders bevorzugt, worin Az für 1H-1,2,4-Triazol steht; Ar unsubstituiertes oder bevorzugt in 2- und/oder 4-Stellung durch Methyl oder Halogen vorzugsweise Fluor oder Chlor, substituiertes Phenyl oder Phenoxyphenyl bedeutet; $R_1$ für Wasserstoff steht; $R_2$ Wasserstoff, Fluor oder Methyl bedeutet; und $R_3$ für Wasserstoff, Fluor, $C_1$—$C_4$-Alkyl oder einen durch Fluor, Chlor und/oder Brom substituierten Rest aus der Reihe Phenyl, Phenoxy und Phenylthio steht.

Fungizid besonders bevorzugte Einzelsubstanzen sind beispielsweise:

1-(1H-1,2,4-Triazol-1-yl)-2-(2,4-dichlorphenyl)-3-fluorbutan-2-ol,
1-(1H-1,2,4-Triazol-1-yl)-2-(2-chlor-4-fluorphenyl)-3-fluorbutan-2-ol,
1-(1H-1,2,4-Triazol-1-yl)-2-(2,4-dichlorphenyl)-3-fluorpentan-2-ol,
1-(1H-1,2,4-Triazol-1-yl)-2-(2,4-dichlorphenyl)-3-fluor-4-methylpentan-2-ol,
1-(1H-1,2,4-Triazol-1-yl)-2-(2-chlor-4-fluorphenyl)-3-fluorpentan-2-ol,
1-(1H-1,2,4-Triazol-1-yl)-2-(2,4-dichlorphenyl)-3-(4-chlorphenoxy)-3-fluorpropan-2-ol,
1-(1H-1,2,4-Triazol-1-yl)-2-[p-(4-chlorphenoxy)phenyl]-3-fluorpropan-2-ol,
1-(1H-1,2,4-Triazol-1-yl)-2-(4-chlorphenyl)-3-fluorhexan-2-ol,
1-(1H-1,2,4-Triazol-1-yl)-2-(2,4-dichlorphenyl)-3-fluorhexan-2-ol,
1-(1H-1,2,4-Triazol-1-yl)-2-(2,4-dichlorphenyl-3,3-difluorpentan-2-ol,
1-(1H-1,2,4-Triazol-1-yl)-2-(2,4-dichlorphenyl)-3-fluor-4-methylpentan-2-ol,
1-(1H-1,2,4-Triazol-1-yl)-2-[p-(4-bromphenoxy)phenyl]-3,3-difluorpropan-2-ol,
1-(1H-1,2,4-Triazol-1-yl)-2-[p-(4-fluorphenoxy)phenyl]-3,3-difluorpropan-2-ol,
1-(1H-1,2,4-Triazol-1-yl)-2-[p-(4-chlorphenoxy)phenyl]-3,3-difluorpropan-2-ol,
1-(1H-1,2,4-Triazol-1-yl)-2-[p-(4-chlorphenoxy)-2-methylphenyl]-2-hydroxy-3-fluorpropan.

Die Verbindungen der Formel I werden dadurch hergestellt, dass man ein Oxiran der Formel II

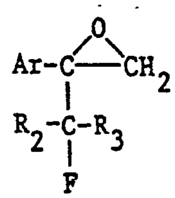

(II)

mit einem Azol der Formel III

M—Az (III)

zuerst zu einer Verbindung der Formel Ia

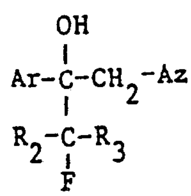

(Ia)

umsetzt, und sofern erforderlich den Alkohol Ia auf übliche Weise, z.B. durch Reaktion mit einer Verbindung der Formel IV

$R_1$—W (IV)

in einen Ether der Formel I überführt, wobei die Substituenten $R_1$, $R_2$, $R_3$, Ar und Az in den Formeln Ia, II, III und IV die unter Formel I angegebenen Bedeutungen haben, M für Wasserstoff oder bevorzugt ein Metallatom, insbesondere ein Alkalimetallatom wie Li, Na oder K steht und W für OH oder eine übliche Abgangsgruppe steht. Uebliche Abgangsgruppen sind aus der Literatur bekannt.

Die Reaktion II mit III zu Ia wird gegebenenfalls in Gegenwart von Kondensationsmitteln oder von säurebindenden Mitteln durchgeführt. Als solche kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, KOH, NaH, $Ca(OH)_2$, $KHCO_3$, $NaHCO_3$, $Ca(HCO_3)_2$, $K_2CO_3$, $Na_2CO_3$), sowie Alkaliacetate wie $CH_3COONa$ oder $CH_3COOK$. Darüberhinaus eignen sich auch Alkalialkoholate wie $C_2H_5ONa$, $C_3H_7$-nONa usw. In einigen Fällen kann es von Vorteil sein, wenn man das freie Azol III (M = Wasserstoff) zuerst, z.B. in situ mit einem Alkoholat, in das entsprechende Salz überführt und anschliessend mit dem Oxiran der Formel II umsetzt. Bei der Herstellung der 1,2,4-Triazolderivate entstehen im allgemeinen parallel auch 1,3,4-Triazolylisomere, die sich auf übliche Weise, z.B. mit unterschiedlichen Lösungsmitteln, voneinander trennen lassen.

Die Reaktion (II mit III zu Ia) wird bevorzugt in einem relativ polaren, jedoch reaktionsinerten organischen Lösungsmittel, durchgeführt, z.B. N,N-Dimethylformamid, N,N-Dimethylacetamid, Dimethylsulfoxid, Acetonitril, Benzonitril und anderen. Derartige Lösungsmittel können in Kombination mit anderen reaktionsinerten Lösungsmitteln, z.B. Benzol, Toluol, Xylol, Hexan, Petrolether, Chlorbenzol, Nitrobenzol u.a. verwendet werden. Die Reaktionstemperaturen liegen in einem Temperaturbereich von 0° bis 150°C, vorzugsweise 20° bis 100°C.

Im übrigen kann diese Reaktion (II mit III zu Ia) analog zu bereits bekannten Umsetzungen von anderen Oxiranen mit Azolen [vgl. DE—OS 29 12 288] durchgeführt werden.

Bei den genannten Teilreaktionen können die Zwischenprodukte aus dem Reaktionsmedium isoliert und falls gewünscht, vor der Weiterreaktion, auf eine der allgemein üblichen Methoden gereinigt werden, z.B. durch Waschen, Digerieren, Extraktion, Kristallisation, Chromatographie, Destillation usw.

Die Weiterreaktion von Ia zu I erfolgt in den Fällen, in denen W in Formel IV für eine übliche Abgangsgruppe steht, in Abwesenheit oder bevorzugt in Anwesenheit eines reaktionsinerten Lösungsmittels.

Es eignen sich z.B. folgende Lösungsmittel: N,N-Dimethylformamid, N,N-Dimethylacetamid, Hexamethylphosphortriamid, Dimethylsulfoxid, 2-Methyl-2-pentanon, usw. Auch Gemische dieser Lösungsmittel untereinander oder mit anderen üblichen inerten organischen Lösungsmitteln, z.B. mit aromatischen Kohlenwasserstoffen wie Benzol, Toluol, Xylolen usw. können verwendet werden. In manchen Fällen kann es sich als vorteilhaft erweisen, zur Beschleunigung der Reaktionsgeschwindigkeit in Gegenwart einer Base wie z.B. eines Alkalimetallhydrides, -hydroxides oder -carbonates zu arbeiten. Es kann aber auch von Vorteil sein, dass man den Alkohol der Formel Ia ($R_1$ = OH) zuerst auf an sich bekannte Weise, z.B. durch Reaktion mit einer starken Base, in ein geeignetes Metallsalz überführt.

Geeignete starke Basen sind z.B. Alkali- und Erdalkalihydride (NaH, KH, $CaH_2$ usw.) und Alkaliorganische Verbindungen wie z.B. Butyllithium oder Alkali-tert.-Butoxid, darüberhinaus können auch Alkalihydroxide, wie NaOH oder KOH eingesetzt werden, wenn man in einem wässrigen Zweiphasensystem und in Anwesenheit eines Phasentransferkatalysators arbeitet.

Man kann jedoch auch den Alkohol der Formel Ia, vor der Weiterreaktion zuerst auf übliche Weise in ein Alkalialkoholat überführen und dann mit einer Verbindungen der Formel IV (worin W für eine Abgangsgruppe steht) umsetzen, dabei arbeitet man vorteilhafterweise in Gegenwart eines Kronenethers. Bei M = K, insbesondere in Gegenwart von 18 Krone-6; bei M = Na, insbesondere in Gegenwart von 15 Krone-5. Dabei wird die Reaktion zweckmässigerweise in einem reaktionsinerten Medium durchgeführt. Als Lösungsmittel eignen sich z.B. Ether und etherartige Verbindungen, z.B. Diniederalkylether (Diethylether, Diisopropylether, tert.-Butylmethylether usw.), Tetrahydrofuran, Dioxan und aromatische Kohlenwasserstoffe wie Benzol, Toluol oder Xylole.

Für die organische, mit Wasser nicht mischbare Phase kommen dabei z.B. folgende Lösungsmittel in Frage: Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylmethylether usw. Beispiele geeigneter Phasentransfer-Katalysatoren sind: Tetraalkylammoniumhalogenide, -hydrogensulfate oder -hydroxide wie Tetrabutylammoniumchlorid, -bromid, -jodid; Triethylbenzylammoniumchlorid, -bromid; Tetrapropylammoniumchlorid, -bromid, -jodid; usw. Als Phasentransfer-Katalysatoren kommen auch Phosphonium-Salze in Betracht. Die Reaktionstemperaturen liegen im allgemeinen zwischen 30° und 130°C, bzw. am Siedepunkt des Lösungsmittels oder Lösungsmittelgemisches.

In den Fällen, in denen W in Formel IV für Hydroxygruppen stehen, wird vorteilhafterweise eine Kondensationsreaktion durchgeführt. Beide Reaktanden werden in einem geeigneten Lösungsmittel unter Rückfluss erhitzt.

Hierbei können grundsätzlich Lösungsmittel eingesetzt werden, die sich gegenüber den Reaktionspartnern inert verhalten und zweckmässigerweise mit Wasser Azeotrope bilden. Es eignen sich hierzu beispielsweise aromatische Kohlenwasserstoffe, wie Benzol, Toluol, Xylole oder halogenierte Kohlenwasserstoffe, wie Dichlormethan, Chloroform, Tetrachlorkohlenstoff, 1,2-Dichloräthan, Tetrachloräthylen, Chlorbenzol, aber auch ätherartige Verbindungen, wie tert.-Butylmethyläther, Dioxan und andere. In manchen Fällen kann die Verbindung der Formel III selbst als Lösungsmittel verwendet werden. Bei dieser Kondensationsreaktion arbeitet man zweckmässigerweise in Gegenwart einer starken

Säure, z.B. Paratoluolsulfonsäure und bei Siedetemperaturen der azeotropen Mischung.

Zur Herstellung der Ether der Formel I kann man auch die freie OH-Gruppe in den Verbindungen der Formel Ia, zuerst gegen eine der obengenannten, üblichen Abgangsgruppen W austauschen und anschliessend mit einer Verbindung der Formel IV (mit W = OH) umsetzen.

Die Ausgangsprodukte der Formel III sind allgemein bekannt oder lassen sich nach an sich bekannten Methoden herstellen.

Die Oxirane der Formel II sind neu, sie stellen besonders entwickelte Zwischenprodukte zur Herstellung der wertvollen Wirkstoffe der Formel I dar. Aufgrund ihrer strukturellen Beschaffenheit lassen sie sich in einfacher Weise in die Verbindungen der Formel Ia überführen, darüberhinaus zeigen Verbindungen der Formel II zum Teil fungizide Aktivität gegenüber Schadpilzen aus den Familien Ascomycetes, Basidiomycetes oder Fungi imperfecti.

Epoxide der Formel II können in an sich bekannter Weise aus Ketonen der Formel V

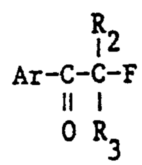

(V)

durch Umsetzung mit Dimethylsulfoniummethylid oder Dimethyloxosulfoniummethylid hergestellt werden (Corey and Chaykovsky, JACS, 1962, *84*, 3782).

Die Ketone der Formel V sind nach an sich aus der Literatur bekannten Methoden [vgl. J. Leroy, J. Org. Chem. *46*, 206 (1981) oder Houben-Weyl, Band V/3, S. 211] aus den entsprechenden bekannten α-Brom-Ketonen durch üblichen Austausch des Broms gegen Fluor zugänglich oder können auch durch Acylierung des zugrundeliegenden Aromaten mit fluorierten Carbonsäurederivaten z.B. nach Friedel-Crafts hergestellt werden.

Bei der Herstellung aller hierin genannten Ausgangs-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon oder in gewissen Fällen auch Kohlendioxid.

Die Verbindungen der Formel I

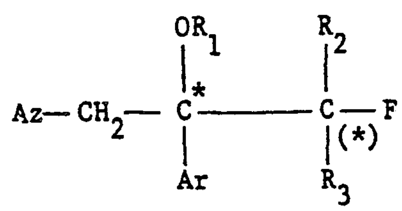

(I)

besitzen in Nachbarstellung zu den Substituenten Ar und $OR_1$ stets ein asymmetrisches C-Atom C* und können daher in zwei enantiomeren Formen vorliegen. Im allgemeinen ensteht bei der Herstellung dieser Substanzen ein Gemisch beider Enantiomerer, dieses lässt sich auf übliche Weise, z.B. durch fraktionierte Kristallisation von Salzen mit optisch aktiven, starken Säuren, in die reinen optischen Antipoden aufspalten. Die Enantiomeren können unterschiedliche biologische Wirkungen aufweisen, so kann z.B. bei der einen Form die fungizide und bei der anderen die pflanzenregulatorische Wirkung im Vordergrund stehen. Auch kann bei gleichem Wirkungsspektrum ein gradueller Aktivitätsunterschied auftreten. Sind die Reste $R_2$ und $R_3$ verschieden, so tritt ein weiteres Asymmetrie-Zentrum (*) auf, was zur Existenz von diastereomeren Gemischen führt (threo- und erythro-Formen), die mittels physikalischer Methoden getrennt werden können.

Die vorliegende Erfindung betrifft alle reinen Enantiomeren bzw. Diastereomeren und deren Gemische untereinander.

Das beschriebene Herstellungsverfahren ist, einschliesslich aller Teilschritte, ein wichtiger Bestandteil der vorliegenden Erfindung.

Es wurde überraschend festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe der Formel I sind gegen die den folgenden Klassen angehörenden phytopathogenen Pilze wirksam: Fungi imperfecti (z.B. Botrytis, Helminthosporium, Fusarium, Septoria, Cercospora und Alternaria); Basidiomyceten (z.B. die Gattungen Hemileia, Rhizocotonia, Puccinia); insbesondere wirken sie gegen die Klasse der Ascomyceten (z.B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkuturen für die hierin offenbarten Indikationsgebiete gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bacterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B.

mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylcholin, Sphingomyelin, Phosphatidylinosit, Phosphatidylglycerin, Lysolecithin, Plasmalogene oder Cardiolipin, die man beispielsweise aus tierischen oder pflanzlichen Zellen, insbesondere aus Hirn, Herz, Leber, Eidotter oder Sojabohnen gewinnen kann. Verwendbare Handelsmischungen sind z.B. Phosphatidylcholin-Mischungen. Synthetische Phospholipide sind z.B. Dioctanoylphosphatidylcholin und Dipalmitoylphosphatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$—$C_{22}$) genannt, wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürliche Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8—22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4—14)-Ethylenoxyd-Adduktes in Frage.

Als nichtionische Tenside kommen in erster Linie Polyglykoletherderivative von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 g Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxyaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"McCutcheon's Detergents and Emulsifiers Annual" BC Publishing Corp., Ridgewood New Jersey, 1981.

Helmut Stache "Tensid-Taschenbuch" Carl Hauser-Verlag München/Wien 1981.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet: h = Stunde; d = Tag; min = Minute; RT = Raumtemperatur; N = Normalität; abs = absolut, wasserfrei; DMSO = Dimethylsulfoxid; DMF = Dimethylformamid. Druckangaben erfolgen in Millibar mb, oder Bar b.

HERSTELLUNGSBEISPIELE

Beispiel H1

Herstellung von

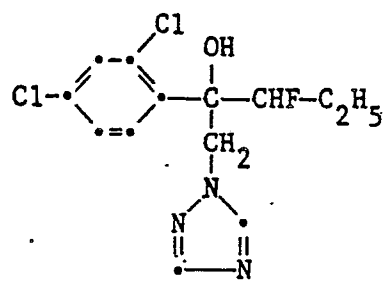

1-(1H-1,2,4-Triazol-1-yl)-2-(2,4-dichlorphenyl)-3-fluor-pentan-2-ol

a) *Herstellung der Vorstufe*

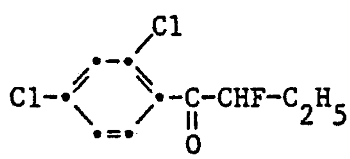

1-(2,4-Dichlorphenyl)-2-fluor-butanon

Zu einer Mischung von 77 g 1-(2,4-Dichlorphenyl)-2-brombutanon und 500 mg 18-Krone-6 in 750 ml absolutem Acetonitril gab man 31 g trockenes Kaliumfluorid und erhitzte das Gemisch langsam unter Rühren auf 100° bis 110°C. Nach ca. 48 Stunden war die Reaktion beendet (Kontrolle gaschromatographisch oder durch NMR). Die Reaktionslösung wurde nun auf 2 Liter Eiswasser gegossen und mehrmals mit Diethylether extrahiert. Die vereinigten Extrakte wurden mit Wasser gewaschen, über Natriumsulfat getrocknet und eingedampft. Ausbeute 57 g des öligen Produkts. [H—F Kopplungs-Konstante 50 Hz] Sdp. 77—78°/0,008 mbar.

b) *Herstellung einer weiteren Vorstufe*

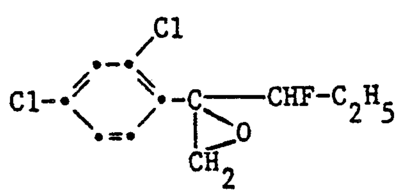

2-(2,4-Dichlorphenyl)-2-(1-fluorpropyl)-oxiran

8 g 80 %iges Natriumhydrid wurde in 300 ml absolutem DMSO aufgeschlämmt. Dazu wurden unter Stickstoffatmosphäre 68 g Trimethyloxosulfoniumjodid unter Rühren portionsweise eingetragen. Nach Beendigung der Wasserstoffentwicklung und nach Abklingen der exothermen Reaktion wurde das

Gemisch noch 2 Stunden bei RT gerührt. Dann wurde eine Lösung von 57 g 1-(2,4-Dichlorphenyl)-2-fluorbutanon in 100 ml THF innerhalb von 30 Minuten zugetropft, das resultierende Gemisch 3 Stunden gerührt und anschliessend mit Eiswasser auf das fünffache Volumen verdünnt und mehrmals mit Diethylether extrahiert. Die vereinigten Extrakte wurden mit Wasser gewaschen, über Natriumsulfat getrocknet und in Vakuum von Lösungsmittel befreit. Ausbeute 55 g in Form eines braunen Oels.

c) *Herstellung des Endproduktes*

Eine Mischung von 55 g 2-(2,4-Dichlorphenyl)-2-(1-fluorpropyl)-oxiran, 30 g 1,2,4-Triazol und 3,5 g Kalium-tert.-butylat in 500 ml DMF wurden 20 Stunden bei 80°C gerührt. Danach wurde die Reaktionslösung auf RT abgekühlt, auf 2 Liter Eiswasser gegossen, mehrfach mit Diethylether extrahiert. Die vereinigten Extrakte wurden mit Wasser gewaschen, über Natriumsulfat getrocknet und eingeengt. Ausbeute an 1-(1H-1,2,4-Triazol-1-yl)-2-(2,4-dichlorphenyl)-3-fluorpentan-2-ol 26 g in Form von farblosen Kristallen. Smp. 204—206°C.

Beispiel H2

Herstellung von

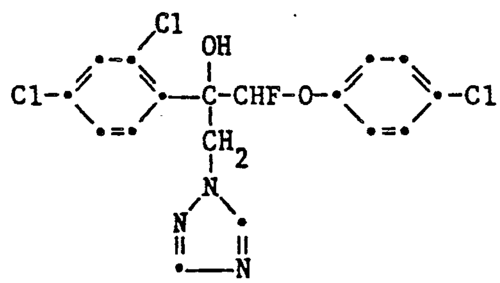

1-(1H-1,2,4-Triazol-1-yl)-2-(2,4-dichlorphenyl)-3-(4-chlorphenoxy)-3-fluorpropan-2-ol

a) *Herstellung der Vorstufe*

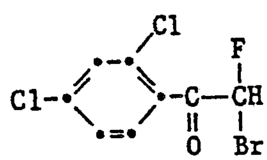

1-(2,4-Dichlorphenyl)-2-brom-2-fluorethanon

Zu einer Lösung von 20,7 g α-Fluor-2,4-dichloracetophenon in 100 ml Tetrachlorkohlenstoff wurde bei 40° bis 45°C eine Lösung von 16 g Brom in 100 ml Tetrachlorkohlenstoff zugegeben. Nach ca. 1 Stunde hatte sich die braune Lösung entfärbt. Es wurde noch 1 Stunde weitergerührt und anschliessend mit wässriger Natriumhydrogencarbonatlösung ausgeschüttelt und im Vakuum eingedampft. Der ölige Rückstand wurde dann im Hochvakuum destilliert. Ausbeute 17 g. Sdp. 89—92°C/0,02 mbar.

b) *Herstellung einer weiteren Vorstufe*

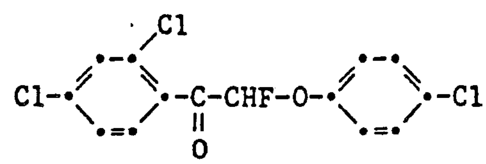

1-(2,4-Dichlorphenyl)-2-(4-chlorphenoxy)-2-fluorethanon

12,8 g Chlorphenol und 13,8 g Kaliumcarbonat wurden in 200 ml Aceton 1 Stunde gerührt. Zu dieser Suspension wurden 28 g 1-(2,4-Dichlorphenyl)-2-brom-2-fluorethanon in 50 ml Aceton zugetropft und das Gemisch 3 Stunden unter Rückfluss erhitzt. Nach dem Abkühlen auf RT wurde der farblose Salzniederschlag abfiltriert, das Aceton im Vakuum entfernt und Diethylether zugesetzt. Die Etherlösung wurde mit Wasser gewaschen, über Natriumsulfat getrocknet, filtriert und eingeengt. Das ölige Rohprodukt kristallisiert nach dem Digerieren mit n-Hexan. Ausbeute 21,5 g in Form von gelblichen Kristallen. Smp. 85—87°C.

c) *Herstellung einer weiteren Vorstufe*

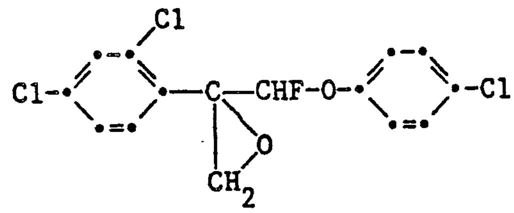

2-(2,4-Dichlorphenyl)-2-(4-chlorphenoxyfluormethyl)-oxiran

1 g 80 %iges Natriumhydrid wurde unter Stickstoffatmosphäre in 80 ml DMSO gerührt und portionsweise mit 10,3 g Trimethyloxosulfoniumjodid versetzt. Nach dem Abklingen der exothermen Reaktion wurde das Gemisch noch 1 Stunde bei RT gerührt, dann wurde eine Lösung von 2-(2,4-Dichlorphenyl)-2-(4-chlorphenoxy)-2-fluorethanon in 30 ml THF zugetropft, die resultierende Mischung 5 Stunden bei 25° bis 30°C weitergerührt und anschliessend auf 1 Liter Wasser gegossen. Das Produkt wurde mit Diethylether extrahiert, die Extrakte mit Wasser gewaschen, über Natriumsulfat getrocknet, filtriert und eingeengt. Ausbeute 15 g als gelbliches Oel.

d) *Herstellung des Endproduktes*

Eine Lösung von 13 g 2-(2,4-Dichlorphenyl)-2-(4-chlorphenoxyfluormethyl)-oxiran, 4 g 1,2,4-Triazol und 0,5 g Kalium-tert.-butylat in 100 ml DMF wurde 15 Stunden bei 80° bis 100°C gerührt. Nach dem Abkühlen auf RT wurde die Reaktionslösung in 500 ml Wasser gegossen, wobei sich das rohe Produkt als Oel abschied. Es wurde mit Diethylether extrahiert, die vereinigten Extrakte mit Wasser gewaschen, über Natriumsulfat getrocknet, filtriert und eingeengt. Ausbeute 11 g eines öligen Rohproduktes, das beim Digerieren mit n-Hexan kristallisierte. Ausbeute des gereinigten Produktes 7 g. Smp. 155—157°C.

Auf analoge Weise lassen sich auch die nachfolgend aufgeführten Substanzen herstellen:

Tabelle 1: Verbindungen der Formel

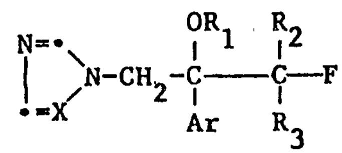

| Verb. Nr. | Ar | $R_1$ | $R_2$ | $R_3$ | X | Physik. Konst. |
|---|---|---|---|---|---|---|
| 1 | $C_6H_3Cl_2(2,4)$ | H | H | 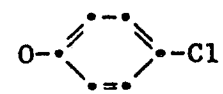 | N | Smp. 155-157° |
| 2 | $C_6H_3Cl_2(2,4)$ | H | H | $C_2H_5$ | N | Smp. 204-206° |
| 3 | $C_6H_3Cl_2(2,4)$ | H | H | H | N | Smp. 142-143° |
| 4 | $C_6H_4Cl(4)$ | H | H | $C_3H_7$-n | N | Smp. 149-151° |
| 5 | $C_6H_3Cl(2)F(4)$ | H | H | H | N | Smp. 132-133° |
| 6 | 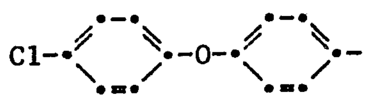 | H | H | H | N | Smp. 118-120° |
| 7 | 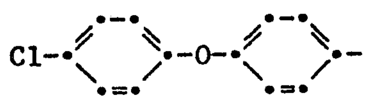 | H | H | H | CH | Smp. 156-157° |
| 8 | $C_6H_3Cl(2,4)$ | H | H. | $CH_3$ | N | Smp. 202-204° |

Tabelle 1 (Fortsetzung)

| Verb. Nr. | Ar | $R_1$ | $R_2$ | $R_3$ | X | Physik. Konst. |
|---|---|---|---|---|---|---|
| 9 | 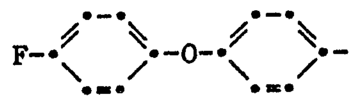 | H | H | H | N | Smp. 87-100° |
| 10 | 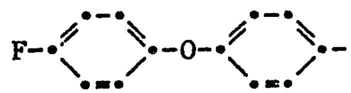 | H | H | H | CH | Smp. 100-105° |
| 11 | $C_6H_4F(4)$ | H | F | F | N | Smp. 128-130° |
| 12 | $C_6H_3Cl_2(2,4)$ | $CH_3$ | H | $C_2H_5$ | N | |
| 13 | $C_6H_3Cl_2(2,4)$ | Benzyl | H | H | N | |
| 14 | $C_6H_3Cl(2)F(4)$ | H | H | $C_2H_5$ | N | Smp. 171-173° |
| 15 | 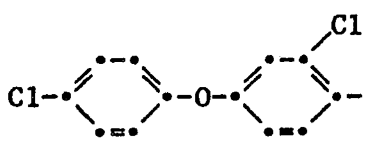 | H | H | H | N | Smp. 160-163° |
| 16 | 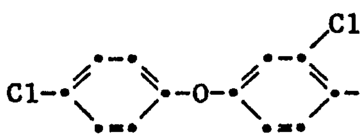 | H | H | H | CH | |
| 17 | $C_6H_3Cl(2)F(4)$ | $CH_2CH{=}CH_2$ | H | $C_2H_5$ | N | Harz |

Tabelle 1 (Fortsetzung)

| Verb. Nr. | Ar | $R_1$ | $R_2$ | $R_3$ | X | Physik. Konst. |
|---|---|---|---|---|---|---|
| 18 | $C_6H_3Cl_2(2,4)$ | H | H | $CH_2-CH(CH_3)_2$ | N | |
| 19 | $C_6H_3Cl_2(2,4)$ | H | H | $CH(CH_3)_2$ | N | Smp. 175 - 176° |
| 20 | $C_6H_4F(4)$ | 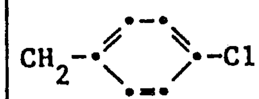 | H | H | N | Harz |
| 21 | $C_6H_4F(4)$ | 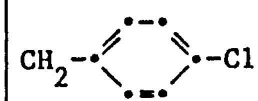 | H | H | CH | |
| 22 | $C_6H_4F(4)$ | H | H | $CH_2CH_2Cl$ | N | |
| 23 | $C_6H_4F(4)$ | H | H | 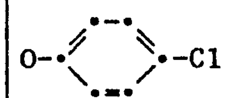 | N | |
| 24 | $C_6H_3Cl_2(2,4)$ | H | $CH_3$ | 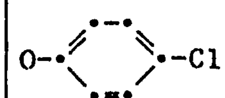 | N | |
| 25 | $C_6H_3Cl_2(2,4)$ | $CH_3$ | H | $CH_3$ | N | Harz |
| 26 | $C_6H_4Br(4)$ | H | H | $C_2H_5$ | N | |
| 27 | $C_6H_4Br(4)$ | $CH_3$ | H | $C_2H_5$ | N | |

Tabelle 1 (Fortsetzung)

| Verb. Nr. | Ar | $R_1$ | $R_2$ | $R_3$ | X | Physik. Konst. |
|---|---|---|---|---|---|---|
| 28 | Biphenyl-4-yl | H | H | $CH_3$ | N | |
| 29 | $C_6H_3Cl_2(2,4)$ | H | H | $OC_2H_5$ | N | |
| 30 | $C_6H_3Cl_2(2,)$ | H | H | $SC_3H_7$-n | N | |
| 31 | $C_6H_4Cl(2)$ | H | H | $OCH_3$ | N | |
| 32 | $C_6H_3Cl(2)F(4)$ | H | H | 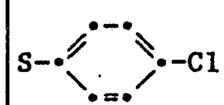 | N | |
| 33 | $C_6H_3Cl(2)F(4)$ | H | H | $CH_3$ | N | Smp. 163-165° |
| 34 | 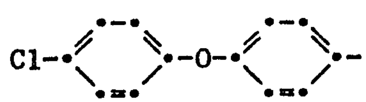 | H | H | $CH_3$ | CH | Smp. 146-147° |
| 35 | 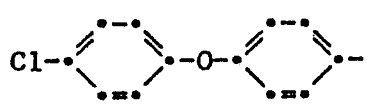 | H | H | $CH_3$ | N | Smp. 173-174° |

Tabelle 1 (Fortsetzung)

| Verb. Nr. | Ar | $R_1$ | $R_2$ | $R_3$ | X | Physik. Konst. |
|---|---|---|---|---|---|---|
| 36 | $C_6H_3Cl_2(2,4)$ | H | H | $C_4H_9$-n | N | |
| 37 | $C_6H_4Cl(4)$ | H | H | $C_6H_4Cl(4)$ | N | |
| 38 | $C_6H_4Cl(2)F(4)$ | H | H | $CH_3$ | N | Smp. 186-198° |
| 39 | $C_6H_3Cl(2)F(4)$ | $CH_3$ | H | $CH_3$ | N | |
| 40 | $C_6H_3Cl(2)F(4)$ | H | $CH_3$ | 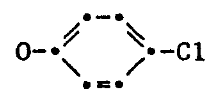 | N | |
| 41 | $C_6H_4Cl_2(2,4)$ | H | F | $C_2H_5$ | N | |
| 42 | $C_6H_3Cl_2(2,4)$ | H | H | $C_2H_5$ | CH | Smp.195-196° |
| 43 | $C_6H_3Cl_2(2,4)$ | H | H | $C_3H_7$-n | N | Smp. 141-142° |
| 44 | $C_6H_3Cl_2(2,4)$ | H | H | $C_4H_9$-n | N | Smp. 101-102° |
| 45 | 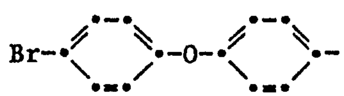 | H | H | H | N | Smp. 118-119° |
| 46 | 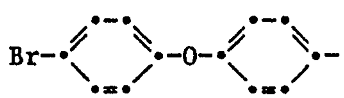 | H | H | H | CH | Smp. 120-125° |
| 47 | $C_6H_4F(4)$ | H | H | $C_2H_5$ | N | Smp. 141-143° |
| 48 | $C_6H_3F_2(2,4)$ | H | H | $C_2H_5$ | N | Smp. 154-156° |
| 49 | 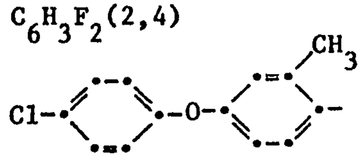 | H | H | H | N | viskoses Oel |

Tabelle 1 (Fortsetzung)

| Verb. Nr. | Ar | $R_1$ | $R_2$ | $R_3$ | X | Physik. Konst. |
|---|---|---|---|---|---|---|
| 50 | 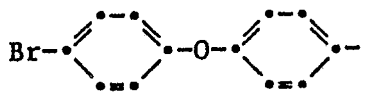 | H | F | H | N | Smp. 130° |
| 51 | 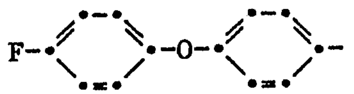 | H | F | H | N | Smp.70-72° |
| 52 | 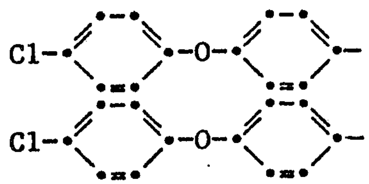 | H | F | H | N | Smp. 125-127° |
| 53 | | $CH_3$ | F | H | N | viskoses Oel |
| 54 | 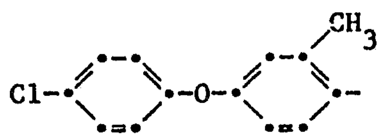 | $CH_3$ | H | $CH_3$ | N | Harz |
| 55 | 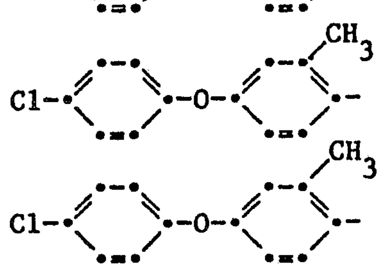 | $CH_2CH{=}CH_2$ | H | $CH_3$ | N | |
| 56 | | H | H | $C_2H_5$ | N | |

Tabelle 1 (Fortsetzung)

| Verb. Nr. | Ar | $R_1$ | $R_2$ | $R_3$ | X | Physik. Konst. |
|---|---|---|---|---|---|---|
| 57 | $C_6H_3Cl_2$(2,4) | H | H | $C_6H_5$ | N | |
| 58 | $C_6H_4Cl$(4) | H | H | $C_6H_5$ | N | |
| 59 | 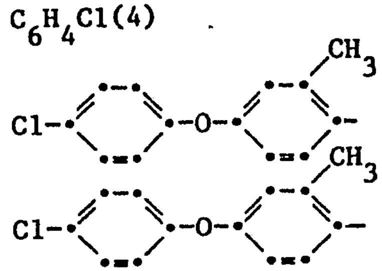 | H | H | $CH_3$ | N | |
| 60 | | H | H | $C_2H_5$ | N | |
| 61 | 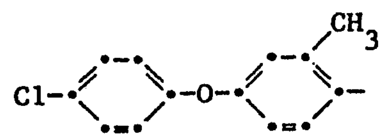 | $CH_2-C_6H_5$ | H | $CH_3$ | N | |
| 62 | 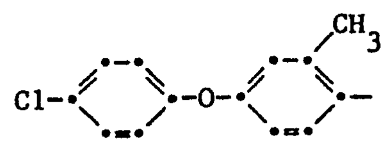 | $CH_2-C_6H_5$ | H | $C_2H_5$ | N | |
| 63 | 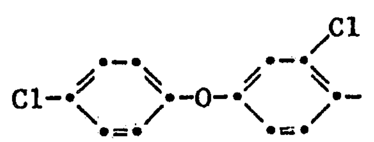 | H | H | $CH_3$ | N | |

Formulierungsbeispiele für flüssige Wirkstoffe der Formel I (% = Gewichtsprozent)

| F1. Emulsions-Konzentrate | a) | b) | c) |
|---|---|---|---|
| Wirkstoff aus der Tabelle | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykolether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus der Tabelle | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| F3. Granulate | a) | b) |
|---|---|---|
| Wirkstoff aus der Tabelle | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus der Tabelle | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| F5. Spritzpulver | a) | b) | c) |
|---|---|---|---|
| Wirkstoff aus der Tabelle | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F6. Emulsions-Konzentrat | |
|---|---|
| Wirkstoff aus der Tabelle | 10% |
| Octylphenolpolyethylenglykolether (4-5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus der Tabelle | 5% | 8% |
| Talkum | 95% | - |
| Kaolin | - | 92% |

Man erhält anwendungsfertige Stäubemittel, indem der Wirkstoff mit den Trägernvermischt auf einer geeigneten Mühle vermahlen wird.

F8. Extruder Granulat

| | |
|---|---|
| Wirkstoff aus der Tabelle | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

F9. Umhüllungs-Granulat

| | |
|---|---|
| Wirkstoff aus der Tabelle | 3% |
| Polyethylenglykol (M G 200) | 3% |
| Kaolin | 94% |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

F10. Suspensions-Konzentrat

| | |
|---|---|
| Wirkstoff aus der Tabelle | 40% |
| Ethylenglykol | 10% |
| Nonylphenolpolyethylenglykolether (15 Mol Ethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## BIOLOGISCHE BEISPIELE

### Beispiel B1

Wirkung gegen Puccinia graminis auf Weizen

a) *Residual-protektive Wirkung*

Weizenpflanzen wurden 6 Tage nach der Aussaat mit einer aus Spritzpulver des Wirkstoffes hergestellten Spritzbrühe (0,02% Aktivsubstanz) besprüht. Nach 24 Stunden wurden die behandelten Pflanzen mit einer Uredosporensuspension des Pilzes infiziert. Nach einer Inkubation während 48 Stunden bei 95—100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

b) *Systemische Wirkung*

Zu Weizenpflanzen wurden 5 Tage nach Aussaat eine aus Spritzpulver des Wirkstoffes hergestellte Spritzbrühe gegossen (0,006% Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95—100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

Verbindungen aus der Tabelle zeigten gegen Puccinia-Pilze eine sehr gute Wirkung. Unbehandelte aber infizierte Kontrolpflanzen zeigten einen Puccinia-Befall von 100%. Unter anderen hemmten die Verbindungen 1 bis 10, 14, 15, 17, 19, 20, 25, 33—35, 41—47 sowie 49—53 den Puccinia-Befall auf 0 bis 5%.

Beispiel B2

Wirkung gegen Cercospora arachidicola auf Erdnusspflanzen Residual-protektive Wirkung

10—15 cm hohe Erdnusspflanzen wurden mit einer aus Spritzpulver der Wirksubstanz hergestellten Spritzbrühe (0,006% Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt.

Die Beurteilung der fungiziden Wirkung erfolgte 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100%), zeigten Erdnusspflanzen, die mit Wirkstoffen aus der Tabelle behandelt wurden, einen stark reduzierten Cercospora-Befall. So verhinderten die Verbindungen 1 bis 9, 14, 19, 20, 25, 33, 38, 41—47 und 51—54 in obigen Versuchen das Auftreten von Flecken fast vollständig (0—10%).

Beispiel B3

Wirkung gegen Erysiphe graminis auf Gerste

a) *Residual-protektive Wirkung*

Ca. 8 cm hohe Gerstenpflanzen wurden mit einer aus Spritzpulver des Wirkstoffes hergestellten Spritzbrühe (0,002% Aktivsubstanz) besprüht. Nach 3—4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

b) *Systemische Wirkung*

Zu ca. 8 cm hohen Gerstenpflanzen wurde eine aus Spritzpulver des Wirkstoffes hergestellte Spritzbrühe gegossen (0,006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestaübt. Die infizierten Gerstenpflanzen wurden in einem Gewächshaus bei ca. 22°C aufgestellt und der Pilzbefall nach 10 Tagen beurteilt.

Verbindungen der Formel I zeigten gute Wirkung gegen Erysiphe-Pilze. Unbehandelte, aber infizierte Kontrollpflanzen zeigten einen Erysiphe-Befall von 100%. Unter anderen Verbindungen aus der Tabelle hemmten die Verbindungen 1 bis 10, 14, 15, 17, 19, 20, 25, 33, 35, 38, 41—47 und 49—54 den Pilzbefall bei Gerste auf 0 bis 5%, insbesondere Verbindung Nr. 2 bewirkte eine vollständige Befallsreduktion.

Beispiel B4

Residual-protektive Wirkung gegen Venturia inaequalis auf Apfeltrieben

Apfelstecklinge mit 10—20 cm langen Frischtrieben wurden mit einer aus Spritzpulver des Wirkstoffes hergestellten Spritzbrühe (0,006% Aktivsubstanz) besprüht. Nach 24 Stunden wurden die behandelten Pflanzen mit einer Konidiensuspension des Pilzes infiziert. Die Pflanzen wurden dann während 5 Tagen bei 90—100% relativer Luftfeuchtigkeit inkubiert und während 10 weiteren Tagen in einem Gewächshaus bei 20—24°C aufgestellt. Der Schorfbefall wurde 15 Tage nach der Infektion beurteilt. Verbindungen 1 bis 6, 8, 9, 14, 17, 19, 20, 33, 41, 43, 45—47 und 49—53 hemmten den Krankheitsbefall auf weniger als 10%. Unbehandelte aber infizierte Kontrolltriebe wurden dagegen 100 %ig befallen.

Beispiel B5

Wirkung gegen Botrytis cinerea auf Bohnen

*Residual-protektive Wirkung*

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95—100% relativer Luftfeuchtigkeit und 21°C erfolgte die Beurteilung des Pilzbefalls. Die Verbindungen aus der Tabelle hemmten in vielen Fällen die Pilzinfektion sehr stark. Bei einer Konzentration von 0,02% erwiesen sich z.B. die Verbindungen 1 bis 6, 8, 9, 14, 15, 20, 25, 33, 35, 41, 43, 45, 46, 47 sowie 49—53 als voll wirksam. Der Krankheitsbefall lag bei 0 bis 8%.

Der Botrytis-Befall unbehandelter aber infizierter Bohnenpflanzen betrug 100%.

Beispiel B6

Wirkung gegen Piricularia oryzae auf Reispflanzen

*Residual-protektive Wirkung*

Reispflanzen wurden nach zweiwöchiger Anzucht mit einer aus Spritzpulver des Wirkstoffes hergestellten Spritzbrühe (0,002% Aktivsubstanz) besprüht. Nach 48 Stunden wurden die behandelten Pflanzen mit einer Konidiensuspension des Pilzes infiziert. Nach 5 Tagen Inkubation bei 95—100% relativer Luftfeuchtigkeit und 24°C wurde der Pilzbefall beurteilt.

Reispflanzen, die mit einer Spritzbrühe behandelt worden sind, die als Aktivsubstanz eine der Verbindungen aus der Tabelle 1, so z.B. Nr. 14 oder 33 enthielt, zeigten im Vergleich zu unbehandelten Kontrollpflanzen (100% Befall) weniger als 10% Pilzbefall.

**Patentansprüche für die Vertragsstaaten: BE CH LI DE FR IT LU NL SE**

1. Verbindungen der allgemeinen Formel I

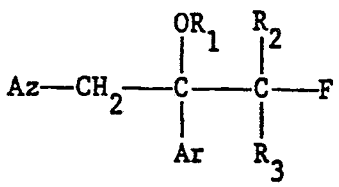

(I)

worin

Az für 1H-1,2,4-Triazol, 4H-1,2,4-Triazol oder 1H-Imidazol steht;

Ar einen unsubstituierten oder substituierten aromatischen Rest aus der Reihe Phenyl, Biphenyl, Phenoxyphenyl und Naphthyl bedeutet;

$R_1$ für Wasserstoff, $C_1$—$C_4$-Alkyl, $C_3$—$C_5$-Alkenyl oder Benzyl steht; und

$R_2$ Wasserstoff, Fluor oder $C_1$—$C_6$-Alkyl bedeutet;

$R_3$ Wasserstoff, Fluor, $C_1$—$C_6$-Alkyl, $C_1$—$C_6$-Haloalkyl, $C_1$—$C_6$-Alkoxy, $C_1$—$C_6$-Alkylthio, Phenyl, Phenoxy, Phenylthio oder $C_3$—$C_7$-Cycloalkyl steht,

wobei jeder aromatische Substituent oder aromatische Anteil eines Substituenten unsubstituiert oder ein- oder mehrfach durch Halogen, $C_1$—$C_4$-Alkyl, $C_1$—$C_4$-Alkoxy, $C_1$—$C_4$-Haloalkyl, Nitro und/oder Cyano substituiert ist, mit der Massgabe, dass $R_2$ und $R_3$ nicht gleichzeitig Fluor sein dürfen; unter Einschluss der Säureadditionssalze, quaternären Azoliumsalze und Metallkomplexe.

2. Eine Verbindung der Formel I gemäss Anspruch 1, ausgewählt aus der Reihe:

1-(1H-1,2,4-Triazol-1-yl)-2-(2,4-dichlorphenyl)-3-fluorbutan-2-ol,
1-(1H-1,2,4-Triazol-1-yl)-2-(2-chlor-4-fluorphenyl)-3-fluorbutan-2-ol,
1-(1H-1,2,4-Triazol-1-yl)-2-(2,4-dichlorphenyl)-3-fluorpentan-2-ol,
1-(1H-1,2,4-Triazol-1-yl)-2-(2,4-dichlorphenyl)-3-fluor-4-methylpentan-2-ol,
1-(1H-1,2,4-Triazol-1-yl)-2-(2-chlor-4-fluorphenyl)-3-fluorpentan-2-ol,
1-(1H-1,2,4-Triazol-1-yl)-2-[p-(4-chlorphenoxy)phenyl]-3-fluorpropan-2-ol,
1-(1H-1,2,4-Triazol-1-yl)-2-(2,4-dichlorphenyl)-3-(4-chlorphenoxy)-3-fluorpropan-2-ol.

3. Eine Verbindung der Formel I gemäss Anspruch 1, ausgewählt aus der Reihe:

1-(1H-1,2,4-Triazol-1-yl)-2-(4-chlorphenyl)-3-fluorhexan-2-ol,
1-(1H-1,2,4-Triazol-1-yl)-2-(2,4-dichlorphenyl)-3-fluorhexan-2-ol,
1-(1H-1,2,4-Triazol-1-yl)-2-(2,4-dichlorphenyl)-3-fluor-4-methylpentan-2-ol.

4. Verfahren zur Herstellung der in Anspruch 1 definierten Verbindungen der Formel I, dadurch gekennzeichnet, dass man ein Oxiran der Formel II

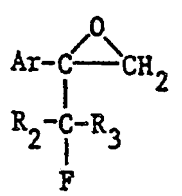

(II)

mit einem Azol der Formel III

M—Az (III)

zuerst zu einer Verbindung der Formel Ia

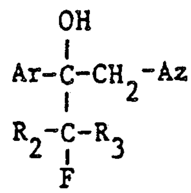

(Ia)

und sofern erforderlich, den Alkohol Ia auf übliche Weise, z.B. durch Reaktion mit einer Verbindung der Formel IV

$$R_1—W \qquad (IV)$$

in einen Ether der Formel I überführt, wobei die Substituenten $R_1$, $R_2$, $R_3$, Ar und Az in den Formeln Ia, II, III und IV die unter Formel I angegebenen Bedeutungen haben, M für Wasserstoff oder ein Metallatom steht und W für OH oder eine übliche Abgangsgruppe steht.

5. Mittel zur Bekämpfung oder Verhütung eines Befalls durch Mikroorganismen, dadurch gekennzeichnet, dass es neben üblichen Trägermaterialen als mindestens eine aktive Komponente eine Verbindung der Formel I gemäss Anspruch 1 enthält.

6. Mittel nach Anspruch 4, dadurch gekennzeichnet, dass es 0,1 bis 99% eines Wirkstoffs der Formel I, 99,9 bis 1% eines festen oder flüssigen Zusatzstoffes und 0 bis 25% eines Tensids enthält.

7. Verfahren zur Bekämpfung oder Verhütung eines Befalls von Kulturpflanzen durch phytopathogene Mikroorganismen, dadurch gekennzeichnet, dass man eine gemäss Anspruch 1 definierte Verbindung der Formel I auf die Pflanze oder deren Standort appliziert.

8. Die Oxirane der Formel II

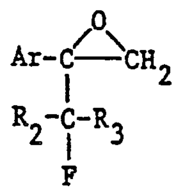

(II)

worin $R_1$, $R_2$, $R_3$ und Ar die in Anspruch 1 unter Formel I angegebenen Bedeutungen haben.

**Patentansprüche für den Vertragsstaat: AT**

1. Mittel zur Bekämpfung oder Verhütung eines Befalls durch Mikroorganismen, dadurch gekennzeichnet, dass es neben üblichen Trägermaterialien oder Zuschlagstoffen als mindestens eine aktive Komponente eine Verbindung der Formel I enthält,

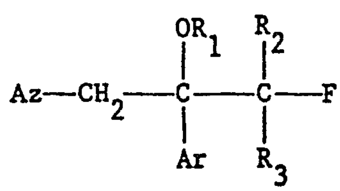

(I)

worin

Az für 1H-1,2,4-Triazol, 4H-1,2,4-Triazol oder 1H-Imidazol steht;

Ar einen unsubstituierten oder substituierten aromatischen Rest aus der Reihe Phenyl, Biphenyl, Phenoxyphenyl und Naphthyl bedeutet;

$R_1$ für Wasserstoff, $C_1$—$C_4$-Alkyl, $C_3$—$C_5$-Alkenyl oder Benzyl steht; und

$R_2$ Wasserstoff, Fluor oder $C_1$—$C_6$-Alkyl bedeutet;

$R_3$ Wasserstoff, Fluor, $C_1$—$C_6$-Alkyl, $C_1$—$C_6$-Haloalkyl, $C_1$—$C_6$-Alkoxy, $C_1$—$C_6$-Alkylthio, Phenyl, Phenoxy, Phenylthio oder $C_3$—$C_7$-Cycloalkyl steht,

wobei jeder aromatische Substituent oder aromatische Anteil eines Substituenten unsubstituiert oder ein- oder mehrfach durch Halogen, $C_1$—$C_4$-Alkyl, $C_1$—$C_4$-Alkoxy, $C_1$—$C_4$-Haloalkyl, Nitro und/oder Cyano substituiert ist, mit der Massgabe, dass $R_2$ und $R_3$ nicht gleichzeitig Fluor sein dürfen; unter Einschluss der Säureadditionssalze, quaternären Azoliumsalze und Metallkomplexe.

2. Mittel nach Anspruch 1, enthaltend als aktive Komponente eine Verbindung der Formel I, ausgewählt aus der Reihe:

1-(1H-1,2,4-Triazol-1-yl)-2-(2,4-dichlorphenyl)-3-fluorbutan-2-ol,

1-(1H-1,2,4-Triazol-1-yl)-2-(2-chlor-4-fluorphenyl)-3-fluorbutan-2-ol,

1-(1H-1,2,4-Triazol-1-yl)-2-(2,4-dichlorphenyl)-3-fluorpentan-2-ol,
1-(1H-1,2,4-Triazol-1-yl)-2-(2,4-dichlorphenyl)-3-fluor-4-methylpentan-2-ol,
1-(1H-1,2,4-Triazol-1-yl)-2-(2-chlor-4-fluorphenyl)-3-fluorpentan-2-ol,
1-(1H-1,2,4-Triazol-1-yl)-2-[p-(4-chlorphenoxy)phenyl]-3-fluorpropan-2-ol,
1-(1H-1,2,4-Triazol-1-yl)-2-(2,4-dichlorphenyl)-3-(4-chlorphenoxy)-3-fluorpropan-2-ol.

3. Mittel nach Anspruch 1, enthaltend als aktive Komponente eine Verbindung der Formel I ausgewählt aus der Reihe:
1-(1H-1,2,4-Triazol-1-yl)-2-(4-chlorphenyl)-3-fluorhexan-2-ol,
1-(1H-1,2,4-Triazol-1-yl)-2-(2,4-dichlorphenyl)-3-fluorhexan-2-ol,
1-(1H-1,2,4-Triazol-1-yl)-2-(2,4-dichlorphenyl)-3-fluor-4-methylpentan-2-ol.

4. Verfahren zur Herstellung der in Anspruch 1 definierten Verbindungen der Formel I, dadurch gekennzeichnet, dass man ein Oxiran der Formel II

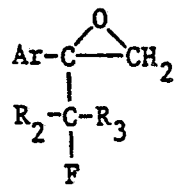

(II)

mit einem Azol der Formel III

$$M—Az \qquad (III)$$

zuerst zu einer Verbindung der Formel Ia

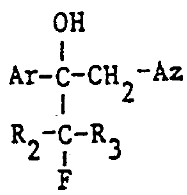

(Ia)

und sofern erforderlich, den Alkohol Ia auf übliche Weise, z.B. durch Reaktion mit einer Verbindung der Formel IV

$$R_1—W \qquad (IV)$$

und sofern erforderlich, den Alkohol Ia auf übliche Weise, z.B. durch Reaktion mit einer Verbindung der Formel IV

$$R_5—W \qquad (IV)$$

in einen Ether der Formel I überführt, wobei die Substituenten $R_1$, $R_2$, $R_3$, Ar und Az in den Formeln Ia, II, III und IV die unter Formel I angegebenen Bedeutungen haben, M für Wasserstoff oder ein Metallatom steht und W für OH oder eine übliche Abgangsgruppe steht.

5. Verfahren zur Herstellung eines agrochemischen Mittel, dadurch gekennzeichnet, dass man mindestens eine gemäss Anspruch 1 definierte Verbindung der Formel I mit geeigneten festen oder flüssigen Zusatzstoffen und Tensiden innig vermischt.

6. Verfahren zur Bekämpfung oder Verhütung eines Befalls von Kulturpflanzen durch phytopathogene Mikroorganismen, dadurch gekennzeichnet, dass man eine gemäss Anspruch 1 definierte Verbindung der Formel I auf die Pflanze oder deren Standort appliziert.

7. Verwendung von Verbindungen der Formel I gemäss Anspruch 1 zur Bekämpfung und/oder präventiven Verhütung eines Befalls von Mikroorganismen.

8. Verwendung gemäss Anspruch 7, dadurch gekennzeichnet, dass es sich bei den Mikroorganismen um phytopathogene Pilze handelt.

9. Verwendung gemäss Anspruch 8 gegen Pilze aus den Klassen Ascomycetes, Basidiomycetes oder Fungi imperfecti.

10. Verwendung der Oxirane der Formel II

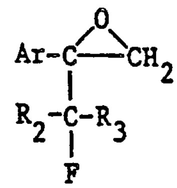

(II)

worin $R_1$, $R_2$, $R_3$ und Ar die in Anspruch 1 unter Formel I angegebenen Bedeutungen haben, zur Herstellung von Mikrobiziden.

**Revendications pour les Etats contractants: BE CH DE FR IT LI LU NL SE**

1. Composés de formule générale I

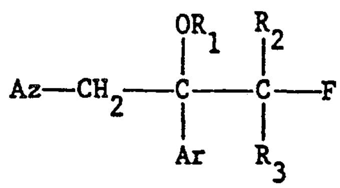

(I)

dans laquelle

Az représente le 1H-1,2,4-triazole, le 4H-1,2,4-triazole ou le 1H-imidazole;

Ar représente un reste aromatique non substitué ou substitué de la série phényle, biphényle, phénoxyphényle et naphtyle;

$R_1$ représente l'hydrogène, un groupe alkyle en $C_1$—$C_4$, alcényle en $C_3$—$C_5$ ou benzyle; et

$R_2$ représente le fluor, l'hydrogène ou un groupe alkyle en $C_1$—$C_6$;

$R_3$ représente l'hydrogène, le fluor, un groupe alkyle en $C_1$—$C_6$, halogénoalkyle en $C_1$—$C_6$, alcoxy en $C_1$—$C_6$, alkylthio en $C_1$—$C_6$, phényle, phénoxy, phénylthio ou cycloalkyle en $C_3$—$C_7$,

chaque substituant aromatique ou partie aromatique d'un substituant étant non substitué ou substitué une ou plusieurs fois par des halogènes, des groupes alkyle en $C_1$—$C_4$, alcoxy en $C_1$—$C_4$, halogénoalkyle en $C_1$—$C_4$, nitro et/ou cyano, sous réserve que $R_2$ et $R_3$ ne peuvent représenter tous deux, simultanément, le fluor; y compris les sels formés par addition avec des acides, sels d'azolium quaternaire et complexes métalliques.

2. Un composé de formule I selon la revendication 1, choisi dans la série des composés suivants:
1-(1H-1,2,4-triazole-1-yl)-2-(2,4-dichlorophényl)-3-fluorobutane-2-ol,
1-(1H-1,2,4-triazole-1-yl)-2-(2-chloro-4-fluorophényl)-3-fluorobutane-2-ol,
1-(1H-1,2,4-triazole-1-yl)-2-(2,4-dichlorophényl)-3-fluoropentane-2-ol,
1-(1H-1,2,4-triazole-1-yl)-2-(2,4-dichlorophényl)-3-fluoro-4-méthyl-pentane-2-ol,
1-(1H-1,2,4-triazole-1-yl)-2-(2-chloro-4-fluorophényl)-3-fluoropentane-2-ol,
1-(1H-1,2,4-triazole-1-yl)-2-[p-(4-chlorophénoxy)phényl]-3-fluoropropane-2-ol,
1-(1H-1,2,4-triazole-1-yl)-2-(2,4-dichlorophényl)-3-(4-chlorophénoxy)-3-fluoropropane-2-ol.

3. Un composé de formule I selon la revendication 1, choisi parmi les suivants:
1-(1H-1,2,4-triazole-1-yl)-2-(4-chlorophényl)-3-fluorohexane-2-ol,
1-(1H-1,2,4-triazole-1-yl)-2-(2,4-dichlorophényl)-3-fluorohexane-2-ol,
1-(1H-1,2,4-triazole-1-yl)-2-(2,4-dichlorophényl)-3-fluoro-4-méthyl-pentane-2-ol.

4. Procédé de préparation des composés de formule I définis dans la revendication 1, caractérisé en ce que l'on convertit d'abord un oxiranne de formule II

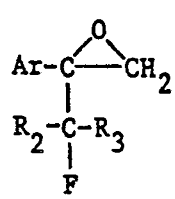

(II)

à l'aide d'un azole de formule III

$$M—Az \qquad (III)$$

en un composé de formule Ia

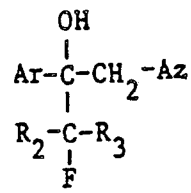

(Ia)

et lorsque c'est nécessaire, on convertit l'alcool Ia de la manière habituelle, par exemple par réaction avec un composé de formule IV

$$R_1—W \qquad (IV)$$

en un éther de formule I, les symboles $R_1$, $R_2$, $R_3$, Ar et Az ayant, dans les formules Ia, II, III et IV les significations indiquées en référence à la formule I, M représentant l'hydrogène ou un atome de métal et W OH ou un groupe éliminable usuel.

5. Produit pour combattre ou prévenir une infection par des microorganismes, caractérisé en ce qu'il contient, avec des véhicules usuels, au moins un composant actif consistant en un composé de formule I selon la revendication 1.

6. Produit selon la revendication 4, caractérisé en ce qu'il contient de 0,1 à 99% d'une substance active de formule I, de 99,9 à 1% d'un additif solide ou liquide et de 0 à 25% d'un agent tensio-actif.

7. Procédé pour combattre ou prévenir une infection de végétaux cultivés par des microorganismes phytopathogènes, caractérisé en ce que l'on applique un composé de formule I défini dans la revendication 1 sur la plante ou son habitat.

8. Les oxirannes de formule II

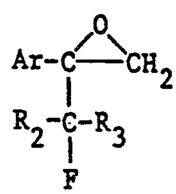

(II)

dans laquelle $R_1$, $R_2$, $R_3$ et Ar ont les significations indiquées dans la revendication 1 en référence à la formule I.

**Revendications pour l'Etat contractant: AT**

1. Produit pour combattre ou prévenir une infection par des microorganismes, caractérisé en ce qu'il contient, avec des véhicules ou additifs usuels, au moins un composant actif consistant en un composé de formule I

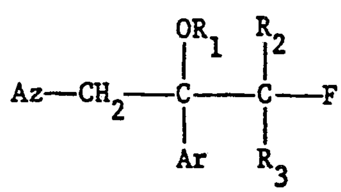

(I)

dans laquelle

Az représente le 1H-1,2,4-triazole, le 4H-1,2,4-triazole ou le 1H-imidazole;

Ar représente un reste aromatique non substitué ou substitué de la série phényle, biphényle, phénoxyphényle et naphtyle;

$R_1$ représente l'hydrogène, un groupe alkyle en $C_1—C_4$, alcényle en $C_3—C_5$ ou benzyle; et

$R_2$ représente l'hydrogène, le fluor ou un groupe alkyle en $C_1—C_6$;

$R_3$ représente l'hydrogène, le fluor, un groupe alkyle en $C_1—C_6$, halogénoalkyle en $C_1—C_6$, alcoxy en $C_1—C_6$, alkylthio en $C_1—C_6$, phényle, phénoxy, phénylthio ou cycloalkyle en $C_3—C_7$,

chaque substituant aromatique ou partie aromatique d'un substituant étant non substitué ou substitué une ou plusieurs fois par des halogènes, des groupes alkyle en $C_1—C_4$, alcoxy en $C_1—C_4$, halogénoalkyle en $C_1—C_4$, nitro et/ou cyano, sous réserve que $R_2$ et $R_3$ ne peuvent représenter tous deux, simultanément, le fluor; y compris les sels formés par addition avec des acides, sels d'azolium quaternaire et complexes métalliques.

2. Produit selon la revendication 1, contenant en tant que composant actif un composé de formule I choisi parmi les suivants:

1-(1H-1,2,4-triazole-1-yl)-2-(2,4-dichlorophényl)-3-fluorobutane-2-ol,
1-(1H-1,2,4-triazole-1-yl)-2-(2-chloro-4-fluorophényl)-3-fluorobutane-2-ol,
1-(1H-1,2,4-triazole-1-yl)-2-(2,4-dichlorophényl)-3-fluoropentane-2-ol,
1-(1H-1,2,4-triazole-1-yl)-2-(2,4-dichlorophényl)-3-fluoro-4-méthyl-pentane-2-ol,
1-(1H-1,2,4-triazole-1-yl)-2-(2-chloro-4-fluorophényl)-3-fluoropentane-2-ol,
1-(1H-1,2,4-triazole-1-yl)-2-[p-(4-chlorophénoxy)phényl]-3-fluoropropane-2-ol,
1-(1H-1,2,4-triazole-1-yl)-2-(2,4-dichlorophényl)-3-(4-chlorophénoxy)-3-fluoropropane-2-ol.

3. Produit selon la revendication 1 contenant en tant que composant actif un composé de formule I choisi parmi les suivants:
1-(1H-1,2,4-triazole-1-yl)-2-(4-chlorophényl)-3-fluorohexane-2-ol,
1-(1H-1,2,4-triazole-1-yl)-2-(2,4-dichlorophényl)-3-fluorohexane-2-ol,
1-(1H-1,2,4-triazole-1-yl)-2-(2,4-dichlorophényl)-3-fluoro-4-méthyl-pentane-2-ol.

4. Procédé de préparation des composés de formule I définis dans la revendication 1, caractérisé en ce que l'on convertit d'abord un oxiranne de formule II

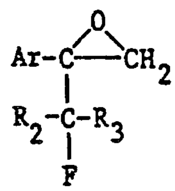

(II)

à l'aide d'un azole de formule III

$$M—Az \qquad (III)$$

en un composé de formule Ia

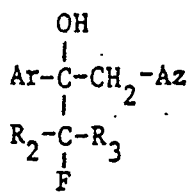

(Ia)

et lorsque c'est nécessaire, on convertit l'alcool Ia de la manière habituelle, par exemple par réaction avec un composé de formule IV

$$R_1—W \qquad (IV)$$

en un éther de formule I, les symboles $R_1$, $R_2$, $R_3$, Ar et Az ayant dans les formules Ia, II, III et IV les significations indiquées en référence à la formule I, M représentant l'hydrogène ou un atome métallique, et W OH ou un groupe éliminable usuel.

5. Procédé de préparation d'un produit agrochimique, caractérisé en ce que l'on mélange intimement au moins un composé de formule I défini dans la revendication 1 avec des additifs solides ou liquides et agents tensio-actifs appropriés.

6. Procédé pour combattre ou prévenir une infection de végétaux cultivés par des microorganismes phytopathogènes, caractérisé en ce que l'on applique un composé de formule I défini dans la revendication 1 sur la plante ou son habitat.

7. Utilisation des composés de formule I selon la revendication 1 pour combattre et/ou prévenir une infection par des microorganismes.

8. Utilisation selon la revendication 7, caractérisée en ce que les microorganismes sont des mycètes phytopathogènes.

9. Utilisation selon la revendication 8 contre des mycètes des classes ascomycètes, basidiomycètes ou Fungi imperfecti.

10. Utilisation des oxirannes de formule II

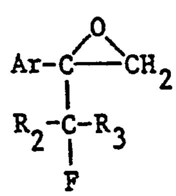

(II)

dans laquelle $R_1$, $R_2$, $R_3$ et Ar ont les significations indiquées dans la revendication 1 en référence à la formule I, pour la préparation de microbicides.

**Claims for the Contracting States: BE CH LI DE FR IT LU NL SE**

1. A compound of the general formula I

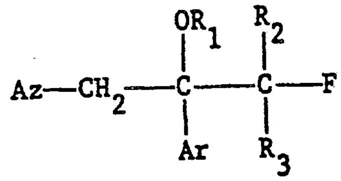

(I)

wherein

Az is 1H-1,2,4-triazole, 4H-1,2,4-triazole or 1H-imidazole;

Ar is an unsubstituted or substituted aromatic radical selected from the group consisting of phenyl, biphenyl, phenoxyphenyl and naphthyl;

$R_1$ is hydrogen, $C_1$—$C_4$alkyl, $C_3$—$C_5$alkenyl or benzyl;

$R_2$ is hydrogen, fluoro or $C_1$—$C_6$alkyl;

$R_3$ is hydrogen, fluoro, $C_1$—$C_6$alkyl, $C_1$—$C_6$haloalkyl, $C_1$—$C_6$alkoxy, $C_1$—$C_6$alkylthio, phenyl, phenoxy, phenylthio or $C_3$—$C_7$cycloalkyl,

each aromatic substituent or aromatic moiety of a substituent being unsubstituted or substituted by one or more of halogen, $C_1$—$C_4$alkyl, $C_1$—$C_4$alkoxy, $C_1$—$C_4$haloalkyl, nitro and/or cyano, with the proviso that $R_2$ and $R_3$ may not simultaneously be fluoro; or an acid addition salt, quaternary azolium salt or metal complex thereof.

2. A compound of formula I according to claim 1 selected from the group consisting of:

1-(1H-1,2,4-triazol-1-yl)-2-(2,4-dichlorophenyl)-3-fluorobutan-2-ol,

1-(1H-1,2,4-triazol-1-yl)-2-(2-chloro-4-fluorophenyl)-3-fluorobutan-2-ol,

1-(1H-1,2,4-triazol-1-yl)-2-(2,4-dichlorophenyl)-3-fluoropentan-2-ol,

1-(1H-1,2,4-triazol-1-yl)-2-(2,4-dichlorophenyl)-3-fluoro-4-methylpentan-2-ol,

1-(1H-1,2,4-triazol-1-yl)-2-(2-chloro-4-fluorophenyl)-3-fluoropentan-2-ol,

1-(1H-1,2,4-triazol-1-yl)-2-[p-(4-chlorophenoxy)phenyl]-3-fluoropropan-2-ol,

1-(1H-1,2,4-triazol-1-yl)-2-(2,4-dichlorophenyl)-3-(4-chlorophenoxy)-3-fluoropropan-2-ol.

3. A compound of formula I according to claim 1 selected from the group consisting of:

1-(1H-1,2,4-triazol-1-yl)-2-(4-chlorophenyl)-3-fluorohexan-2-ol,

1-(1H-1,2,4-triazol-1-yl)-2-(2,4-dichlorophenyl)-3-fluorohexan-2-ol,

1-(1H-1,2,4-triazol-1-yl)-2-(2,4-dichlorophenyl)-3-fluoro-4-methylpentan-2-ol.

4. A process for the preparation of a compound of formula I as defined in claim 1, which comprises converting an oxirane of formula II

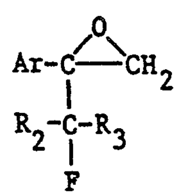

(II)

with an azole of formula III

M—Az (III)

first to a compound of formula Ia

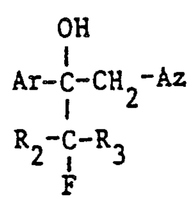

(Ia)

and, if necessary, converting the alcohol of formula Ia, in conventional manner, for example by reaction with a compound of formula IV

$R_1—W$ (IV)

into an ether of formula I, in which formulae Ia, II, III and IV above the substituents $R_1$, $R_2$, $R_3$, Ar and Az are as defined for formula I, M is hydrogen or a metal atom, and W is OH or a customary leaving group.

5. A composition for controlling microorganisms or for preventing infestation by said microorganisms, which contains as at least one active component a compound of formula I as claimed in claim 1, in addition to conventional carriers.

6. A composition according to claim 4, which contains 0.1 to 99% of a compound of formula I, 99.9 to 1% of a solid or liquid carrier, and 0 to 25% of a surfactant.

7. A method of controlling phytopathogenic microorganisms or of preventing infestation of cultivated plants by said microorganisms, which comprises applying to said plants or to the locus thereof a compound of formula I as defined in claim 1.

8. An oxirane of formula II

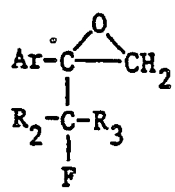

(II)

wherein $R_1$, $R_2$, $R_3$ and Ar are as defined for formula I in claim 1.

**Claims for the Contracting State: AT**

1. A composition for controlling microorganisms or for preventing infestation by said microorganisms, which contains, in addition to conventional carriers, as at least one active component a compound of formula I as claimed in claim 1

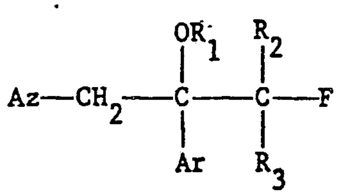

(I)

wherein

Az is 1H-1,2,4-triazole, 4H-1,2,4-triazole or 1H-imidazole;

Ar is an unsubstituted or substituted aromatic radical selected from the group consisting of phenyl, biphenyl, phenoxyphenyl and naphthyl;

$R_1$ is hydrogen, $C_1$—$C_4$alkyl, $C_3$—$C_5$alkenyl or benzyl;

$R_2$ is hydrogen, fluoro or $C_1$—$C_6$alkyl;

$R_3$ is hydrogen, fluoro, $C_1$—$C_6$alkyl, $C_1$—$C_6$haloalkyl, $C_1$—$C_6$alkoxy, $C_1$—$C_6$alkylthio, phenyl, phenoxy, phenylthio or $C_3$—$C_7$cycloalkyl,

each aromatic substituent or aromatic moiety of a substituent being unsubstituted or substituted by one or more of halogen, $C_1$—$C_4$alkyl, $C_1$—$C_4$alkoxy, $C_1$—$C_4$haloalkyl, nitro and/or cyano, with the proviso that $R_2$ and $R_3$ may not simultaneously be fluoro; or an acid addition salt, quaternary azolium salt or metal complex thereof.

2. A composition according to claim 1, which contains as active component a compound of formula I selected from the group consisting of:

1-(1H-1,2,4-triazol-1-yl)-2-(2,4-dichlorophenyl)-3-fluorobutan-2-ol,
1-(1H-1,2,4-triazol-1-yl)-2-(2-chloro-4-fluorophenyl)-3-fluorobutan-2-ol,
1-(1H-1,2,4-triazol-1-yl)-2-(2,4-dichlorophenyl)-3-fluoropentan-2-ol,
1-(1H-1,2,4-triazol-1-yl)-2-(2,4-dichlorophenyl)-3-fluoro-4-methylpentan-2-ol,
1-(1H-1,2,4-triazol-1-yl)-2-(2-chloro-4-fluorophenyl)-3-fluoropentan-2-ol,
1-(1H-1,2,4-triazol-1-yl)-2-[p-(4-chlorophenoxy)phenyl]-3-fluoropropan-2-ol,
1-(1H-1,2,4-triazol-1-yl)-2-(2,4-dichlorophenyl)-3-(4-chlorophenoxy)-3-fluoropropan-2-ol.

3. A composition according to claim 1, which contains as active component a compound of formula I selected from the group consisting of:

1-(1H-1,2,4-triazol-1-yl)-2-(4-chlorophenyl)-3-fluorohexan-2-ol,
1-(1H-1,2,4-triazol-1-yl)-2-(2,4-dichlorophenyl)-3-fluorohexan-2-ol,
1-(1H-1,2,4-triazol-1-yl)-2-(2,4-dichlorophenyl)-3-fluoro-4-methylpentan-2-ol.

4. A process for the preparation of a compound of formula I as defined in claim 1, which comprises converting an oxirane of formula II

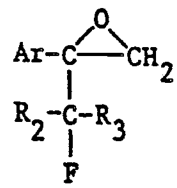

(II)

with an azole of formula III

M—Az (III)

first to a compound of formula Ia

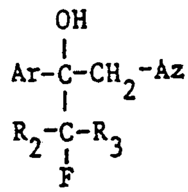

(Ia)

and, if necessary, converting the alcohol of formula Ia, in conventional manner, for example by reaction with a compound of formula IV

$R_1$—W (IV)

into an ether of formula I, in which formulae Ia, II, III and IV above the substituents $R_1$, $R_2$, $R_3$, Ar and Az are as defined for formula I, M is hydrogen or a metal atom, and W is OH or a customary leaving group.

5. A process for the preparation of an agrochemical composition, which comprises intimately mixing at least one compound of formula I as defined in claim 1 with suitable solid or liquid carriers and surfactants.

6. A method of controlling phytopathogenic microorganisms or of preventing infestation of cultivated plants by said microorganisms, which comprises applying to said plants or to the locus thereof a compound of formula I as defined in claim 1.

7. Use of a compound of formula I as claimed in claim 1 for controlling microorganisms and/or for preventing infestation by said microorganisms.

8. Use according to claim 7, wherein the microorganisms are phytopathogenic fungi.

9. Use according to claim 8, wherein the fungi are of the classes Ascomycetes, Basidiomycetes or fungi imperfecti.

10. Use of an oxirane of formula II

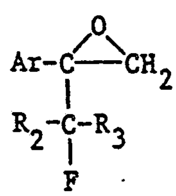

(II)

wherein $R_1$, $R_2$, $R_3$ and Ar are as defined for formula I in claim 1, for the preparation of microbicides.